# DEMANDE DE BREVET EUROPEEN

(11) **EP 0 526 311 A1**
(43) Date de publication de la demande: **03.02.1993**
(21) Numéro de dépôt: 92402123.1
(22) Date de dépôt: 23.07.1992
(51) Int. Cl.: C07D 305/14

(54) **Procédé de préparation de la cinnamoyl-13alpha baccatine III ou désacétyl-10 baccatine III**

(30) Priorité: 25.07.1991 FR 9109424
(71) Demandeur: RHONE-POULENC RORER S.A., 92160 Antony (FR)
(72) Inventeur: de Suzzoni, Sophie, F-91140 Villebon sur Yvette (FR)
(74) Mandataire: Pilard, Jacques

(57) **Abrégé**

Procédé de préparation de la cinnamoyl-13α baccatine III ou désacétyl-10 baccatine III de formule (I) par condensation d'un anhydride mixte de formule générale (II) sur un dérivé de la baccatine III ou de la désacétyl-10 baccatine III de formule générale (III).

Dans les formules (I) et (III), R₁ représente un radical acétyle ou trichloro-2,2,2 éthoxycarbonyle.

Dans la formule (II), R représente 1 à 5 substituants, identiques ou différents (halogène, nitro, méthyle, méthoxy).

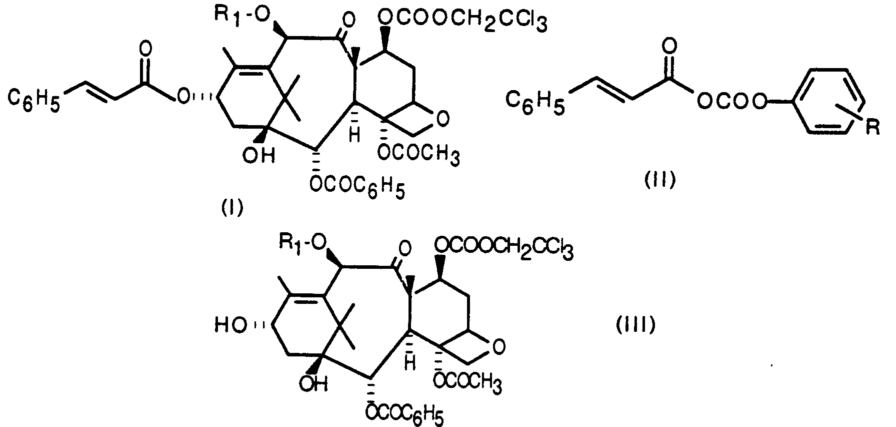

## Description

La présente invention concerne un nouveau procédé de préparation de la cinnamoyl-13α baccatine III ou désacétyl-10 baccatine III de formule générale: 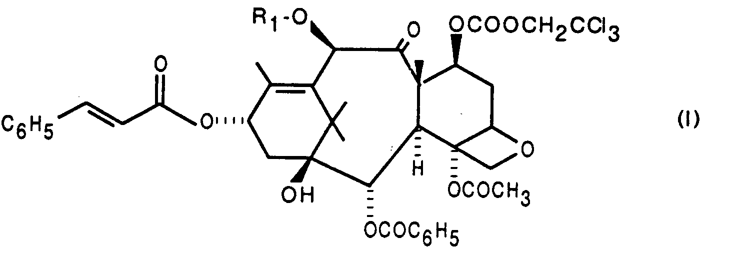
dans laquelle R₁ représente un radical acétyle ou trichloro-2,2,2 éthoxycarbonyle.

Dans le brevet européen EP-0 253 738 a été décrite la préparation du produit de formule générale (I) par action de l'acide cinnamique sur un dérivé de la baccatine III ou de la désacétyl-10 baccatine III de formule générale: 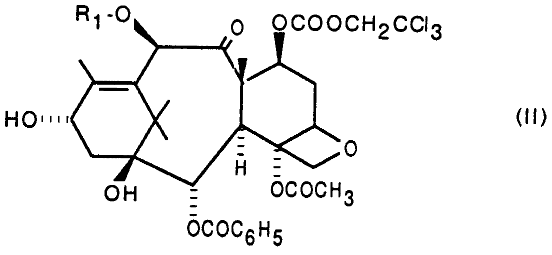
en opérant dans un hydrocarbure aromatique en présence d'un agent de condensation tel qu'un carbodiimide (dicyclohexylcarbodiimide) ou un carbonate réactif (dipyridyl-2 carbonate) et d'un agent d'activation tel que la diméthylaminopyridine à une température comprise entre 6 et 90°C. Pour la mise en oeuvre du procédé il est nécessaire d'utiliser au moins 4 moles d'acide cinnamique par mole de baccatine III ou de désacétyl-10 baccatine III de formule générale (II) pour obtenir des résultats satisfaisants.

Il a maintenant été trouvé, et c'est ce qui fait l'objet de la présente invention que le produit de formule générale (I) peut être obtenu par action d'un anhydride mixte de l'acide cinnamique avec un acide benzoïque substitué sur le dérivé de la baccatine III ou de la désacétyl-10 baccatine III en milieu basique en utilisant une mole d'anhydride mixte par mole de dérivé de la baccatine III ou de la désacétyl-10 baccatine III.

L'anhydride mixte de formule générale : 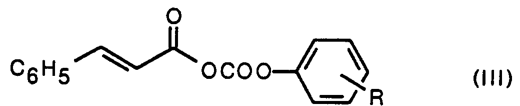
dans laquelle R représente 1 à 5 substituants, identiques ou différents, choisis parmi les atomes d'halogène (chlore, brome) et les radicaux nitro, méthyle ou méthoxy, peut être obtenu par action de l'acide cinnamique sur le chlorure d'acide d'un acide benzoïque de formule générale : 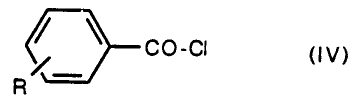
dans laquelle R est défini comme précédemment dans les conditions décrites par J. INANAGA et al., Bull. Chem. Soc. Japan, 52 (7) 1989-1993 (1979). D'un intérêt tout particulier est le chlorure de l'acide trichloro-2,4,6 benzoïque.

Généralement, l'anhydride mixte de formule générale (III) est obtenu par action du chlorure d'acide de formule générale (IV) sur l'acide cinnamique en solution dans un solvant organique convenable tel que le tétrahydrofuranne en présence d'une base organique telle qu'une amine tertiaire comme la triéthylamine à une température comprise entre 0 et 30°C.

De préférence, afin d'éviter les réactions secondaires, la réaction de l'anhydride mixte de formule générale (III) sur le dérivé de la baccatine III ou de la désacétyl-10 baccatine III est effectuée en ajoutant lentement une solution de l'anhydride mixte dans un solvant organique convenable à une solution du dérivé de la baccatine III ou de la désacétyl-10 baccatine III dans le même solvant en présence d'un agent de condensation tel que la pyrrolidinopyridine ou la diméthylaminopyridine. Il est particulièrement avantageux d'opérer sous une atmosphère inerte telle qu'une atmosphère d'argon.

Comme solvant, on utilise de préférence un hydrocarbure aromatique, choisi parmi le benzène, le toluène ou les xylènes, ou un éther tel que le tétrahydrofuranne.

Il est particulièrement avantageux d'opérer à une température comprise entre 40 et 110°C et de préférence à 80°C.

Le produit de formule générale (I) obtenu par le procédé selon la présente invention peut être transformé en produit de formule générale : 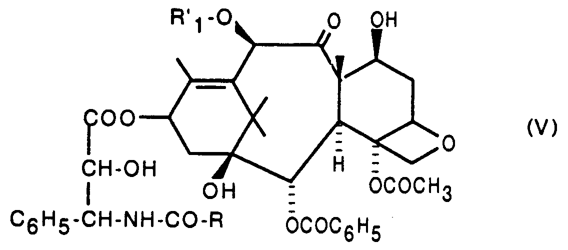
dans laquelle R représente un radical t.butoxy ou phényle et R′₁ représente un atome d'hydrogène ou un radical acétyle selon les procédés décrits dans les demandes de brevet européen EP-A-0 253 738 et EP-A-0 253 739.

L'exemple suivant illustre la présente invention.

### EXEMPLE

A 0,605 g d'acide cinnamique (4 mmoles) en solution dans 10 cm3 de tétrahydrofuranne anhydre, on ajoute 0,580 ml de méthylamine anhydre. On ajoute alors, sous atmosphère d'azote, 0,640 ml de chlorure de trichloro-2,4,6 benzoyle (4,1 mmoles) dans 10 cm3 de tétrahydrofuranne anhydre. On agite pendant 1 heure à une température voisine de 20°C. Le chlorhydrate de triéthylamine est séparé par filtration sur verre fritté. Le tétrahydrofuranne est éliminé sous pression réduite. L'anhydride mixte ainsi obtenu est repris par 50 cm3 de toluène anhydre.

Dans un tricol, on dissout, sous atmosphère d'argon, 4,02 g de bis[(trichloro-2,2,2 éthoxy) carbonyloxy]-7β,10β désacétyl-10 baccatine III (4,48 mmoles) et 1,16 g de pyrrolidinopyridine (7,83 mmoles) dans 50 cm3 de toluène anhydre en chauffant à 80°C. On ajoute alors, goutte à goutte, l'anhydride mixte au moyen d'une ampoule à égalisation de pression. L'évolution de la réaction est suivie par chromatographie en couche mince. Après 3 heures à 80°C, il se forme 70 % d'ester.

La phase toluénique est lavée par de l'acide chlorhydrique N, par de l'hydrogénocarbonate de sodium en solution aqueuse à 2,5 % puis est séchée sulfate de sodium. Après filtration et concentration à sec sous pression réduite, le résidu obtenu est purifié par chromatographie sur silice (0,015-0,025 mm) en éluant par un mélange toluène-éther (95-5 en volumes).

On obtient, avec un rendement de 61,9 %, 2,5 g de cinnamoyl-13α bis[(trichloro-2,2,2 éthoxy) carbonyloxy]-7β,10β désacétyl-10 baccatine III dont les caractéristiques sont identiques à celles qui sont décrites par F. GUERITTE-VOEGELEIN et coll., Tetrahedron, Vol. 42, N° 16, 4451-4460 (1986).

## Revendications

**1 -** Procédé de préparation d'un dérivé du taxane de formule générale : 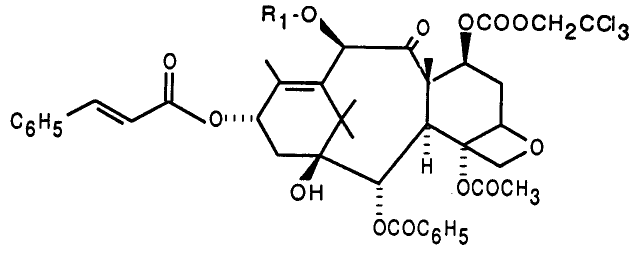 dans laquelle R₁ représente un radical acétyle ou un radical trichloro-2,2,2 éthoxycarbonyle caractérisé en ce que l'on fait réagir un anhydride mixte de formule générale : 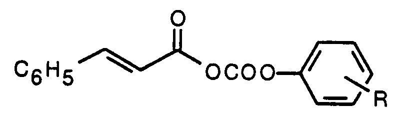 dans laquelle R représente 1 à 5 substituants choisis parmi les atomes d'halogène et les radicaux nitro, méthyle ou méthoxy, sur un dérivé de la baccatine III ou désacétyl-10 baccatine III de formule générale : 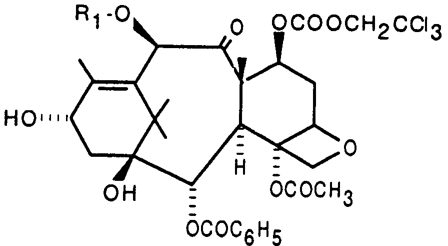 dans laquelle R₁ représente un radical acétyle ou trichloro-2,2,2 éthoxycarbonyle.

**2 -** Procédé selon la revendication 1 caractérisé en ce que l'on opère dans un solvant organique choisi parmi les hydrocarbures aromatiques et les éthers.

**3 -** Procédé selon l'une des revendications 1 ou 2 caractérisé en ce que l'on opère en présence d'une base organique.

**4 -** Procédé selon la revendication 3 caractérisé en ce que la base est choisie parmi la pyrrolidinopyridine ou la diméthylaminopyridine.

**5 -** Procédé selon l'une des revendications 1 à 4 caractérisé en ce que l'on utilise une mole d'anhydride mixte par mole de dérivé de la baccatine III ou de la désacétyl-10 baccatine III.

**6 -** Procédé selon l'une des revendications 1 à 5 caractérisé en ce que l'on opère à une température comprise entre 40 et 110°C.
